# EUROPEAN PATENT APPLICATION

(11) **EP 4 671 736 A1**
(43) Date of publication of application: **31.12.2025**
(21) Application number: 25184960.0
(22) Date of filing: 24.06.2025
(51) Int. Cl.: G01N 21/27, G01N 21/47, G01N 21/898, G01N 33/46

(54) **CALIBRATION METHOD OF AN ANALYSIS INSTRUMENT FOR ANALYSING THE DIRECTION OF WOOD FIBRES OF A PIECE OF WOOD**

(30) Priority: 27.06.2024 IT 202400014908
(71) Applicant: Microtec S.r.l., 39042 Bressanone (BZ) (IT)
(72) Inventor: Silvestri, Luca, 30020 Marcon VE (IT)
(74) Representative: Ponchiroli, Simone

(57) **Abstract**

A calibration method of an analysis instrument (1) for analysing the direction of wood fibres of a piece of wood operating by detecting structured light scattering, wherein a calibration sample (3) is used having a working surface (5) and comprising fibres (17) which can be at least partly illuminated with structured light at the working surface, and wherein a detecting step is carried out, in which a detected trend of the fibres is detected, and a processing step is carried out in which said detected trend of the fibres is processed, and wherein moreover the calibration sample used has the working surface constituted of a composite material (13), which is an anisotropic light diffusion material and which comprises a matrix (15) and said fibres, which are dispersed in the matrix and which have a known trend of the fibres at the working surface.

## Description

This invention relates to a calibration method of an analysis instrument for analysing the direction of wood fibres of a piece of wood.

In the wood industry it is often necessary to identify the direction of the fibres of a piece of wood (for example, of a board) in order to identify knots or other defects, or in general in order to assess the trend of the fibres along the piece of wood, which determines the quality of the piece of wood linked to its structural characteristics and, consequently, its economic value.

To meet that need, the use is known of analysis instruments which measure the direction of the wood fibres at the surface of the piece of wood using a light diffusion phenomenon known in the sector as scattering.

Such analysis instruments operate by irradiating the surface of the piece of wood with one or more structure light beams - movable or fixed - and acquiring images of the surface illuminated in this way. Each beam illuminates a generally circular zone of the surface with a diameter of several tenths of a millimetre, in such a way as to irradiate a limited number of wood fibres whose direction may be considered substantially constant; at the illuminated zone, the light beam generates reflected light and may - in the case of fibres more or less coplanar with the surface - generate light emitted by the piece of wood and diffused mainly along the direction of extension of the fibres at the illuminated zone (emitted light normally illuminates the fibres for lengths of around 2 mm). By detecting the reflected light and any emitted light resulting from scattering (therefore relative to an anisotropic diffusion of the light by the wood), such analysis instruments allow detection of the direction of the illuminated fibres; by performing this type of detecting operation at multiple points of the surface, such instruments allow detection of the trend of the fibres along the piece of wood and identification of any local variations in the direction of the fibres traceable to the defects of the wood (such as knots). For each of these instruments it is routine procedure to periodically perform a calibration of the analysis instrument in order to guarantee the precision of the measurements taken with it. Calibration is used to check the ability of the instrument to take measurements in compliance with the desired performance specifications, by detecting any deviations from the predetermined tolerance limits and determining the quality of the performance of the analysis instrument relative to the specifications, calibration can also be used to determine any instrument corrections required. The calibration may also be accompanied by an adjustment of the analysis instrument, or setting of the instrument (for example in the case of an instrument just manufactured). For these instruments it is also possible to perform a calibration understood as self-calibration (or standardization) before normal use by a user. In the context of this invention, the term calibration is understood to include all of these possibilities.

In order to perform the calibration of the analysis instrument in accordance with the above, the prior art calibration methods involve performing - with the instrument to be calibrated - a set of measurements on a calibration sample constituted of a piece of wood which, at least at a working surface prepared for the measurement, has known characteristics: in particular, that calibration sample has a trend of the surface wood fibres which is known and which is used in the calibration method (the known trend of the fibres is usually ascertained before performing the calibration method by means of measurements of the direction of the fibres with a different instrument with known accuracy). Such calibration methods involve detecting, by means of the analysis instrument to be calibrated, the trend of the fibres at a plurality of points of the working surface of the calibration sample, and then processing the detected trend of the fibres to identify any deviations from the tolerance limits defined by the performance specifications.

However, the prior art has several disadvantages.

In the prior art what is used as the calibration sample is a piece of wood, which is susceptible to modifications - such as for example warping due to environmental conditions (moisture and/or temperature changes) or abrasions of the working surface - which over time may cause the trend of the fibres at the working surface to differ from the originally known trend of the fibres, and which may therefore lead to incorrect calibration of the analysis instrument: processing of the detected trend of the fibres could in fact highlight an incorrect measurement by the instrument even if the analysis instrument were able to correctly measure the direction of the fibres. In order to perform an optimum calibration of the analysis instrument it is therefore necessary to ascertain the trend of the fibres - for example with an instrument having known accuracy - each time the calibration method is carried out; however, that is not always possible, and in any case is complicated.

The use of calibration samples according to the prior art also has critical aspects - intrinsic in the natural origin of the wood and its variability - in terms of reproducibility of the calibration for different exemplars of a same model of analysis instrument which are used in different places (it would be desirable to guarantee the same quality of the calibration for all of the instruments). Therefore, the prior art has several disadvantages, and the technical purpose which forms the basis of this invention is to at least partly overcome them. The technical purpose is substantially achieved by a calibration method as defined in independent claim 1.

Particular embodiments of this invention are defined in the corresponding dependent claims.

Further characteristics and the advantages of this invention will be more apparent from the detailed description of several preferred, non-limiting embodiments of the calibration method.

This description also relates to a calibration sample usable in the calibration method, a use of the calibration sample, and a production method for producing the calibration sample.

Reference will be made to the accompanying drawings, in which:
- Figure 1 is a schematic axonometric view of a detecting step of the calibration method according to this invention with a calibration sample placed in a first position;
- Figure 2 shows the detail II of Figure 1;
- Figure 3 is a schematic axonometric view of a detecting step of the calibration method with the calibration sample placed in a different position to that of Figure 1;
- Figure 4 shows the detail IV of Figure 3;
- Figure 5 is a schematic axonometric view of a detecting step of the calibration method carried out with a different calibration sample;
- Figure 6 shows the detail VI of Figure 5;
- Figure 7 is a schematic axonometric view of an embodiment of the calibration sample;
- Figure 8 shows the detail VIII of Figure 7;
- Figure 9 is an axonometric view of an embodiment of the production method at three different successive moments;
- Figure 10 is an axonometric view of a different embodiment of the production method at four different successive moments.

Below is a description first of the calibration method according to this invention, whilst second and third are descriptions respectively of the calibration sample and the production method for producing the calibration sample. What is described for the calibration method relative to the calibration sample shall also be considered valid, where applicable, for the calibration sample according to this description and vice versa.

The calibration method is a method for calibrating an analysis instrument 1 for analysing the direction of the wood fibres of a piece of wood operating by detecting structured light scattering.

In the calibration method according to this invention, a calibration sample 3 is used having a working surface 5 constituted of a composite material 13. That composite material 13 comprises a matrix 15 (advantageously continuous) and fibres 17, which are dispersed in the matrix 15. At least part of the fibres 17 can be illuminated with structured light at the working surface 5 and, preferably, is located at the working surface 5. In the context of this invention, what are considered possible to illuminate with structured light at the working surface 5 are fibres 17 which can be illuminated with the structured light with which the analysis instrument 1 operates. That means that the fibres 17 may even be covered by the material which constitutes the matrix 15, provided that the thickness and the transmittance of that material allow the structured light with which the working surface 5 is illuminated, to reach them and to come out of the working surface 5 again after having been reflected and/or diffused by the fibres 17. Based on the analysis instrument 1 to be calibrated, the structured light may - by way of example - be light in the visible light band, or light in the near-infra-red band.

At the working surface 5, the fibres 17 have a known trend of the fibres: at each point of the working surface 5 it is possible to identify a limited number of fibres 17 whose direction may be considered constant (more detail on that direction is provided below), and that direction is known point by point at the entire working surface 5 (its trend is known).

The known trend of the fibres may be uniform, or variable: in the former case, the direction of the fibres 17 is constant at the entire working surface 5, whilst in the latter case the direction of the fibres 17 is different at a plurality of separate points of the working surface 5.

Preferably, in the method a calibration sample 3 is used in which the working surface 5 is substantially flat and in which the known trend of the fibres is parallel to the working surface 5, that is to say, in which the direction of the fibres 17 is parallel to the working surface 5 over the entire extent of the working surface 5 (that does not affect the possibility of the known trend of the fibres being uniform or variable).

The composite material 13 is an anisotropic light diffusion material: where "anisotropic light diffusion material" means that the composite material 13, when irradiated with a structured light beam 11 - usually laser light - at the working surface 5, diffuses the light in one direction more than in other directions, similarly to what is observable during irradiation of the wood fibres of a piece of wood with a structured light beam 11.

Using that calibration sample 13, in the calibration method a detecting step and a processing step are carried out.

In the detecting step, carried out with the analysis instrument 1, a detected trend of the fibres is detected at a plurality of points 6 of the working surface 5. Specifically, the detected trend of the fibres is detected by detecting a plurality of detected directions at the plurality of points 6: at each point 6, a structured light beam 11 is used to illuminate illuminated fibres - part of the fibres 17 - and their respective detected direction is detected.

Each detected direction is a direction identified by the analysis instrument 1 relative to a reference system of the instrument: it may, for example, be identified relative to a resting plane 9 on which to rest the calibration sample 3, or relative to a reference direction of the analysis instrument 1 (such as for example a direction of conveying of the pieces of wood).

In some embodiments, the detecting step is a step in which each detected direction is measured relative to that reference system.

In the processing step, taking into account the known trend of the fibres, the detected trend of the fibres is processed to calibrate the analysis instrument 1. The type of processing of the detected trend of the fibres may vary depending on the embodiment of the method.

In some embodiments such as those in which the known trend of the fibres is uniform (in which the direction of the fibres 17 remains constant at the entire working surface 5), in the processing step it is for example possible to check that the detected directions (of the detected trend of the fibres) are identical to each other, or that in any case they differ from each other within predetermined tolerance limits.

In some embodiments, in the detecting step the calibration sample 3 is in a position which is known - or which in any case can be ascertained - relative to the reference system of the analysis instrument 1: in such embodiments it is for example possible, in the processing step, to compare the detected trend of the fibres with the known trend of the fibres by checking, for each point 6 at which detection takes place, the match between the detected direction and the direction of the fibres 17 at that point 6.

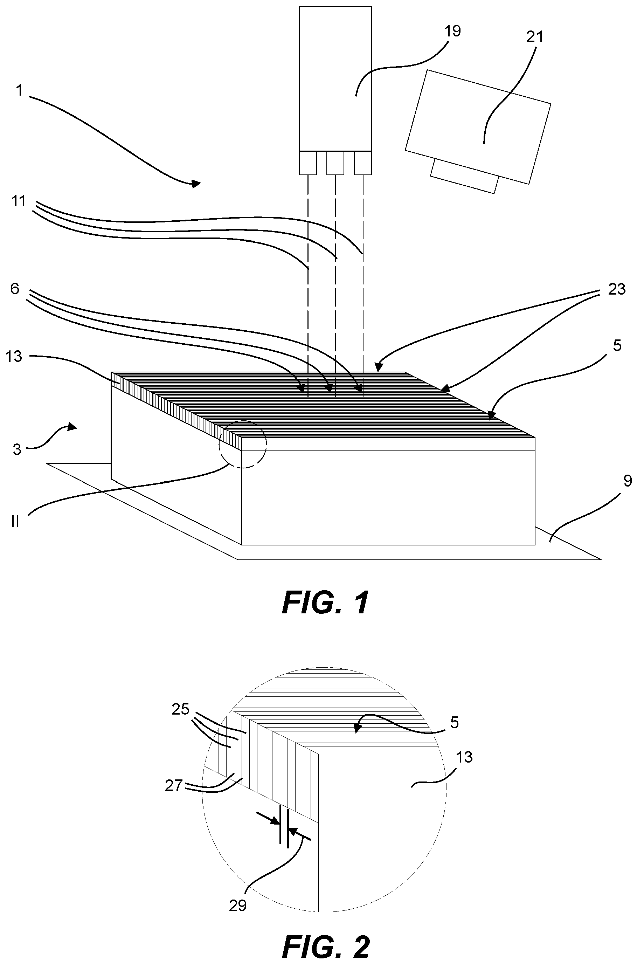

Figure 1 schematically illustrates the detecting step of the calibration method: it shows an emitting device 19 of the analysis instrument 1, a calibration sample 3 placed on a resting plane 9, three fixed structured light beams 11 emitted by the emitting device 19 and incident (in this case perpendicularly) on three points 6 of the working surface 5 of the calibration sample 3, and a detecting device 21 (for example a camera or a video camera) for detecting the reflected light and any light emitted as a result of scattering. The detecting device 21 detects light in the band of frequencies of the light used for the structured light beams 11: for example, if the structured light beams 11 are infra-red light beams, the detecting device 21 is an infra-red camera or video camera. A detail of the calibration sample 3 used is illustrated in Figure 2 (for clarity of illustration, the fibres 17 of the calibration sample 3 are not illustrated).

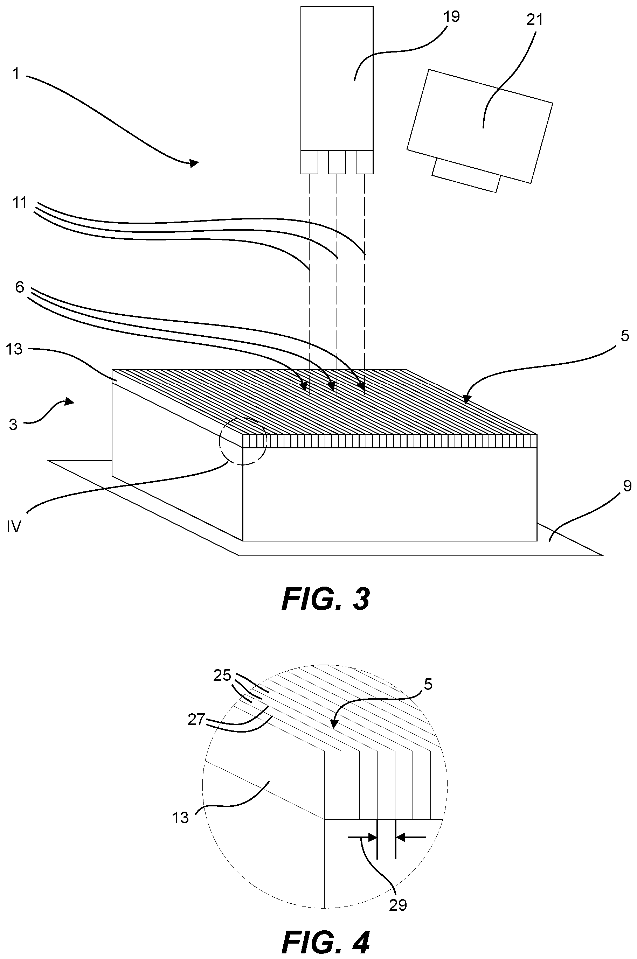

In some embodiments of the calibration method, a plurality of measuring steps are carried out with the calibration sample 3 placed, in each detecting step, in a respective different (known) position relative to the analysis instrument 1: in each detecting step, the detected trend of the fibres is detected at a respective different plurality of points 6 of the working surface 5 and, for each detecting step, the processing step is carried out. Advantageously, in the embodiments in which the known trend of the fibres is uniform, it is possible to check the ability of the analysis instrument 1 to correctly measure directions which are different from each other using the same calibration sample 3. In one possible embodiment, for example, a first detecting step is carried out with the calibration sample 3 in a first position (Figures 1 and 2); then, a second detecting step is carried out with the calibration sample 3 in a second position obtained, starting from the first position, by rotating the calibration sample 3 on the resting plane 9 of the analysis instrument 1 (Figures 3 and 4, in which the calibration sample 3 is rotated 90° relative to Figures 1 and 2).

In some embodiments of the calibration method a plurality of calibration samples 3 are used and, for each calibration sample 3, the detecting step and the processing step are carried out (if necessary a plurality of times with each calibration sample 3 as indicated above). Advantageously, the calibration samples 3 all have the same shape but a different known trend of the fibres.

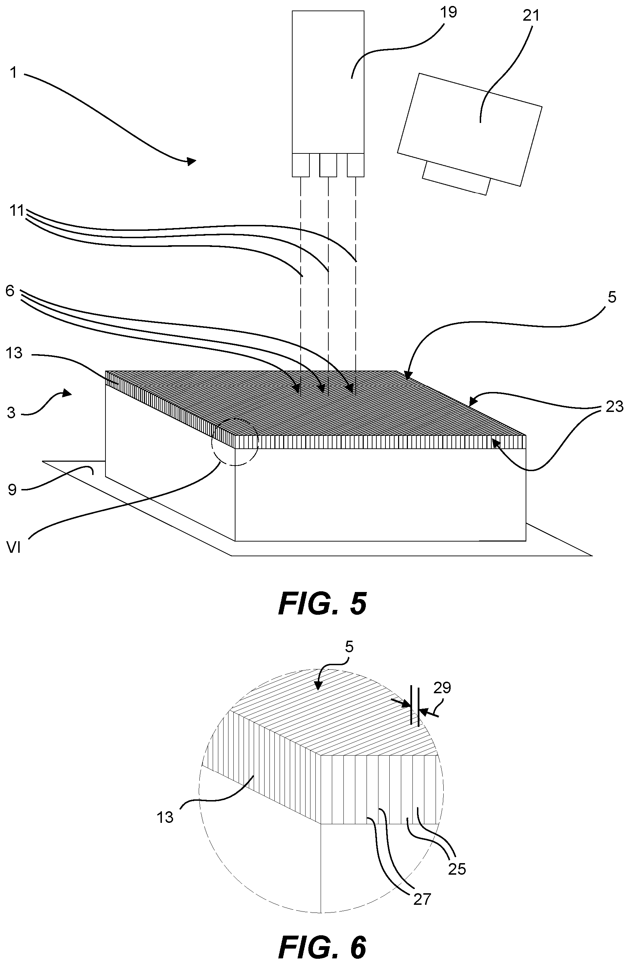

In one possible embodiment, two calibration sample 3 are used having the same parallelepiped shape and a rectangular or square upper face as the working surface 5: the first sample 3 has the fibres 17 with a known trend of the fibres which is parallel to the working surface 5 and which is uniform according to a direction parallel to two edges 23 of the working surface 5 (and perpendicular to another two edges 23); the second sample 3 has the fibres 17 with a known trend of the fibres which is parallel to the working surface 5 and which is uniform according to a direction oblique relative to the four edge 23 of the working surface 5. Reference may be made, for example, to Figures 1 and 2 for the detecting step in which the first sample 3 is used, and to Figures 5 and 6 for the detecting step in which the second sample 3 is used: placing the two calibration samples 3 in the same position relative to the analysis instrument 1, the direction of the fibres 17 of one sample 3 differs by 45° compared with the direction of the fibres 17 of the other sample 3.

Moving on now to a more detailed description of the calibration sample 3 usable in the calibration method just described.

The calibration sample 3 has a working surface 5 constituted of a composite material 13, which is an anisotropic light diffusion material and which comprises a matrix 15 and fibres 17 which are dispersed in the matrix 15; at least part of the fibres 17 can be illuminated with structured light at the working surface 5 and, preferably, is located at the working surface.

The matrix 15 may be a material which is transparent, translucent or even opaque to the structured light with which the analysis instrument 1 operates (at least in the case of an opaque material, the fibres 17 advantageously emerge on the working surface 5). Advantageously, the matrix 15 is made of material having better resistance performance than wood, in particular better resistance to chemical and atmospheric agents.

In preferred embodiments, the matrix 15 is a polymeric or plastic material. Preferably, the polymeric (or plastic) material is made using one or more intrinsically inert polymers (for example, polymers which are hydrophobic by nature and which do not absorb moisture), and/or using additives which, when added to the polymer, give greater resistance (for example, stabilising additives and antioxidant additives).

In other embodiments, the matrix 15 is in contrast a ceramic or metal material. In some preferred embodiments, the matrix 15 of the composite material 13 is PLA-based (polymer known as polylactic acid or polylactide). The matrix 15 may in other cases be ABS-based (acrylonitrile butadiene styrene), HDPE-based (high density polyethylene), polycarbonate-based, or based on other thermoplastic polymers.

In some embodiments, the matrix 15 is thermosetting polymer-based, whilst in yet other embodiments it is elastomeric polymer-based.

Preferably, the matrix 15 is continuous: that is to say, any point inside the matrix 15 can be reached from any other point inside the matrix 15 without exiting the matrix 15.

Regarding the fibres 17, these are dispersed in the matrix 15 and, at least at the working surface 5 they are preferably dispersed as homogeneously as possible.

In the embodiments in which the matrix 15 is a polymeric (or plastic) material, the percentage of fibres 17 by weight in the composite material 13 is advantageously between 5% and 40%, preferably between 20% and 30%. The fibres 17 have a trend of the fibres which is known, and which is parallel to the working surface 5 (the working surface 5 is, advantageously, substantially flat): at each point of the working surface 5 it is possible to identify a limited number of fibres 17 whose direction may be considered constant; that direction of the fibres 17 is known point by point at the entire working surface 5 (its trend is known) and is substantially parallel to the working surface 5 at each point. The direction of the fibres 17 is understood as a direction indicative of the overall orientation of the limited number of fibres 17 at that point. That direction is understood according to the common meaning in the materials sector, in particular in the wood sector.

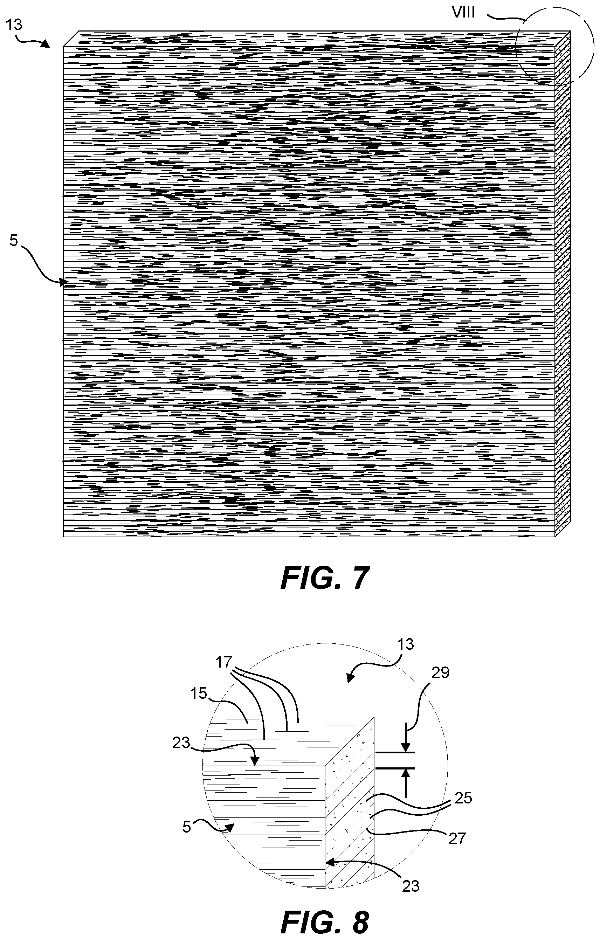

Referring for each fibre 17 to a respective direction of extension parallel to the length of the fibre 17, it is preferably understood that the direction of the fibres 17 at that point is a direction relative to which the directions of extension of at least 90% of the fibres 17 at that point are parallel, or in any case are inclined by an angle of less than 15°, preferably less than 10° and even more preferably less than 5° (the direction of the fibres 17 at that point is in that case an average direction of the main directions of extension of the fibres 17 at that point). Preferably, all of the fibres 17 at the entire working surface 5 are parallel to each other: in that case, the known trend of the fibres is uniform according to a single direction (see for example the calibration sample 3 illustrated in Figure 7 and 8).

With regard to this description, the direction of the fibres 17, like the directions of extension of the fibres 17, and the detected directions are understood as not necessarily having a direction of travel (in the style of a straight line).

In some embodiments, the fibres 17 of the calibration sample 3 are short fibres. In this description short fibres are understood to be fibres 17 having a maximum length to diameter ratio equal to one hundred. Preferably, moreover, the short fibres have a minimum length to diameter ratio equal to three.

In other embodiments, the fibres 17 of the calibration sample 3 in contrast have higher length to diameter ratios.

Preferably, all of the fibres 17 have similar diameters and similar lengths (as illustrated in Figures 7 and 8).

Regarding the materials, the fibres 17 may be of an inorganic type (for example glass fibres) or an organic type (for example polymeric fibres), preferably made of the same material. In some embodiments, the fibres 17 are wood fibres, preferably from wood flour and preferably all of the same plant variety.

In some embodiments, the composite material 13 is an extruded material.

In some embodiments, the composite material 13 has a layered structure, comprising a plurality of layers 25, and the working surface 5 which is transversal to the plurality of layers 25 (see for example the embodiments obtainable with the production method described below). In that layered structure, the fibres 17 are divided in the plurality of layers 25: between one layer 25 and another there are interface zones 27 where fibres 17 which pass from one layer 25 to the other are absent. In some cases, the interface zones 27 have local gaps in the matrix 15.

In some embodiments, each layer 25 has a relative thickness 29 which has an order of magnitude less than or equal to the order of magnitude of the average length of the fibres 17.

In some embodiments, each layer 25 has a relative thickness 29 which is less than or equal to 0.2 mm, even more preferably less than or equal to 0.1 mm. In some embodiments, each layer 25 has a relative thickness 29 which is substantially constant; the thickness 29 of one layer 25 may in any case differ from the thickness 29 of a different layer 25. In those embodiments, the layers 25 are parallel to each other and the known trend of the fibres is uniform at the entire working surface 5. The calibration samples 3 illustrated in the figures have a plurality of layers 25 all having a same constant thickness 29.

In some embodiments of the calibration sample 3, the known trend of the fibres at the working surface 5 is, as said above, variable along the extent of the working surface 5. In some of those embodiments, the composite material 13 has the layered structure and one or more of the layers 25 has a relative thickness 29 which is variable (in the case of multiple layers 25, even differently from one layer 25 to another) along the extent of the relative layer (25).

The relative thickness 29 of each layer 25 is understood in a direction perpendicular to the interface zones 27 between one layer 25 and another at the working surface 5; if the layers 25 each extend in a plane perpendicular to the working surface 5 (case in which each layer 25 has a constant relative thickness 29), that direction is parallel to the working surface 5.

In any case, the relative thickness 29 of each layer 25 is greater than 0.01 mm.

In some embodiments, the calibration sample 3 is constituted of only the composite material 13 (throughout its entire thickness perpendicularly to the working surface 5).

In contrast, in some embodiments, the calibration sample 3 may comprise a first part constituted of the composite material 13 (which defines the working surface 5) and a second part constituted of a different material, which is fixed to the first part and which supports it.

In some embodiments, the calibration sample 3 comprises a frame which supports one or more panels which are each constituted of the composite material 13 and which define the working surface 5 of the calibration sample 3. In some embodiments, the panels are alongside each other at a same side of the calibration sample 3 defining the working surface 5 on that side, whilst in other embodiments those panels are distributed at different sides of the calibration sample 3 (for example at an upper side, at a lower side, at one or more lateral sides of the calibration sample 3). In some embodiments, the panels differ from each other due to a different known trend of the fibres. Although developed for calibrating an analysis instrument 1 for analysing the direction of wood fibres operating by detecting structured light scattering, the calibration sample 3 according to this description may be used for calibrating other wood analysis instruments 1 which carry out optical analysis. Advantageously, the calibration sample 3 may also be used for calibrating wood analysis instruments 1 which carry out a different type of analysis. Finally, the following is a description of the production method for producing the calibration sample 3 described above.

In the production method, the composite material 13 is made by carrying out an extruding step and a depositing step - which is carried out simultaneously with the extruding step - in accordance with a manufacturing method of the Fused Filament Fabrication type, with acronym FFF (known also as Fused Deposition Modelling, with the acronym FDM).

In the extruding step, an extruded material 31 is extruded which comprises a continuous phase and the fibres 17, which are dispersed in the continuous phase. The extruded material 31 may be extruded, for example, starting from a filament, from a bar, or from granules of an extrudable material; the fibres 17 may already be dispersed in advance inside the starting filament, bar, or granules (before carrying out the extruding step), or be dispersed in the continuous phase during extrusion in order to obtain the extruded material 31. The extruded material 31 is extruded along an extruding direction which, advantageously, is vertical. The extruding direction considered may be understood as the axis 32 of the nozzle of an extruding head 33 used for the extruding step.

In the depositing step, the extruded material 31 - coming out along the extruding direction - is deposited along a depositing path forming a plurality of extruded segments 35 superposed on each other in a plurality of layers 25, which extend transversally relative to the working surface 5 of the calibration sample 3 to be made (Figure 7); in particular, each extruded segment 35 is formed along a respective depositing direction (part of the depositing path) which is transversal to the extruding direction.

The depositing direction of each extruded segment 35 corresponds to the direction along which the extruded segment 35 extends lengthwise, and corresponds to the direction of the fibres 17 of the calibration sample 3 to be made along that extruded segment 35.

Advantageously a solidifying step is also carried out in which, every time the extruded material 31 is deposited forming an extruded segment 35, that extruded segment 35 is made to solidify (or left to solidify) before depositing on top of it a different extruded segment 35 (of the plurality of extruded segments 35).

In some embodiments, in the depositing step each extruded segment 35 is formed in such a way that each layer 25 has a relative thickness 29 with an order of magnitude less than or equal to the order of magnitude of the average length of the fibres 17 in order to favour their orientation along the depositing direction.

In some embodiments, in the depositing step, each extruded segment 35 is formed in such a way that each layer 25 has a relative thickness 29 less than or equal to 0.2 mm, even more preferably less than or equal to 0.1 mm.

In some embodiments, in the depositing step, each extruded segment 35 is formed in such a way that each layer 25 has a respective thickness 29 which is constant along an entire length of the extruded segment 35.

**In** any case, each extruded segment 35 is formed in such a way that each layer 25 has a respective height greater than 0.01 mm.

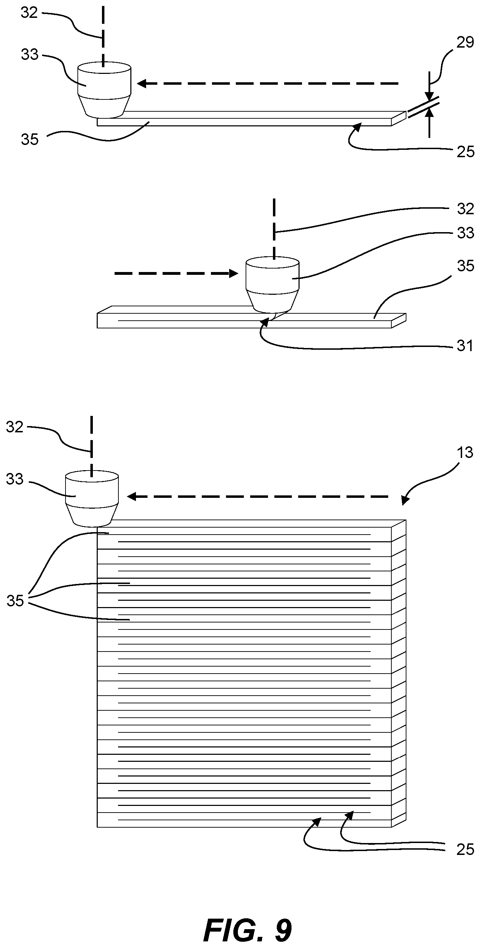

Figure 9 illustrates a possible embodiment of the production method at three different successive moments (from the top down): Figure 9 shows respectively a horizontal first extruded segment 35 just formed, the formation of a second extruded segment 35 on top of the (solidified) first extruded segment 35, and the composite material 13 made as a result of formation of the final extruded segment 35; in the embodiment of Figure 9, the composite material 13 is made with each layer 25 formed by a single extruded segment 35 with constant thickness 29.

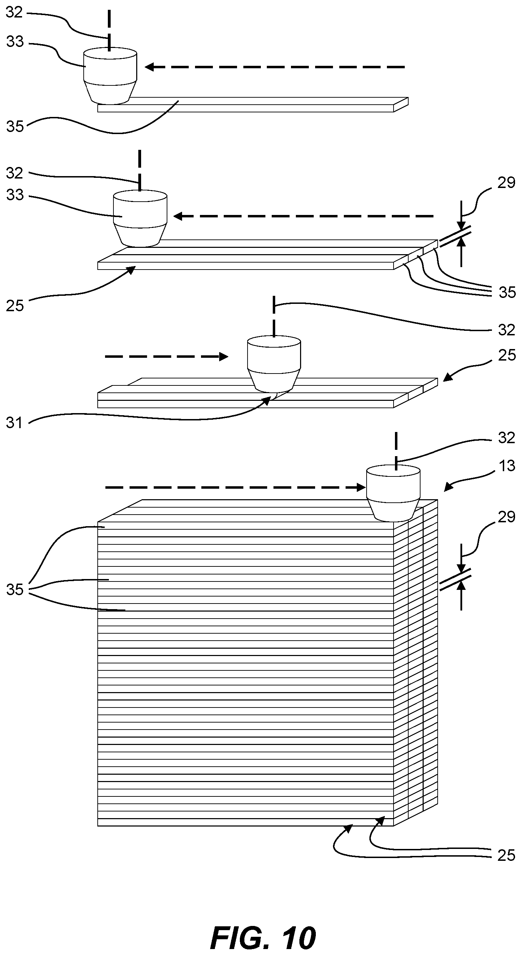

In Figure 9 (and similarly in Figure 10, described below) broken line arrows indicate possible movements of the extruding head 33 during the formation of the extruded segment 35.

In other embodiments, such as that illustrated for example in Figure 10 (illustrated at four different successive moments - from the top down), each layer 25 is in contrast formed by multiple extruded segments 35 (in the case illustrated, three) placed alongside each other in a direction transversal to the depositing direction and to the extruding direction (each extruded segment 35 in any case extends parallel to the depositing direction). For each layer 25, only one extruded segment 35 contributes to defining the working surface 5.

Similarly, in other embodiments, a structure similar to that of Figure 10 may be obtained by making the extruded segments 35 (composed of the extruded material 31) only at the working surface 5 and making the remaining part of the sample using the same manufacturing method of Fused Deposition Modelling but with a different material.

Preferably, for each layer 25 the extruded material 31 is deposited forming, at the working surface 5, a single extruded segment 35 without interrupting the depositing of the extruded material 31. In some cases the entire plurality of extruded segments 35 may be formed without interrupting the depositing of extruded material 31, for example by depositing the extruded material 31 along a winding depositing path (as in the embodiment of Figure 9), or interrupting the depositing of extruded material 31 at the ends of each extruded segment 35 (as in the embodiment of Figure 10).

The production method for producing the calibration sample 3 may comprise further steps: for example, it may comprise a step of cutting the composite material 13 to change the shape and/or the dimensions of the working surface 5 (for example, to obtain a calibration sample 3 with known trend of the fibres which is uniform according to a direction oblique relative to the edges 23 of the working surface 5).

This invention has allowed important advantages to be obtained.

In fact, through this invention it was possible to provide a calibration method which allows the critical aspects linked to the alterability of the pieces of wood used until now as calibration samples to be avoided.

The invention described above may be modified and adapted in several ways without thereby departing from the scope of the inventive concept as defined in the independent claims.

All details may be substituted with other technically equivalent elements and the materials used, as well as the shapes and dimensions of the various components, may vary according to requirements.

## Claims

1. A calibration method of an analysis instrument (1) for analysing the direction of wood fibres of a piece of wood operating by detecting structured light scattering, wherein a calibration sample (3) is used having a working surface (5) and comprising fibres (17) which can be at least partly illuminated with structured light at the working surface (5), and wherein the following steps are carried out:
a detecting step, carried out with the analysis instrument (1), in which a detected trend of the fibres is detected at a plurality of points (6) of the working surface (5), the detection of the detected trend of the fibres being carried out, at each point (6) of the plurality of points (6), using a structured light beam (11) to illuminate illuminated fibres part of the fibres (17) and detecting a respective detected direction of the illuminated fibres; and
a processing step, in which said detected trend of the fibres is processed to calibrate the analysis instrument (1);
and wherein moreover the calibration sample (3) which is used has the working surface (5) constituted of a composite material (13), the composite material (13) being an anisotropic light diffusion material and comprising a matrix (15) and said fibres (17), which are dispersed in the matrix (15) and which have a known trend of the fibres at the working surface (5).

2. The calibration method according to claim 1, **characterised in that** a calibration sample (3) is used in which the known trend of the fibres is parallel to the working surface (5).

3. The calibration method according to claim 1 or 2, **characterised in that**:
a plurality of said detecting steps is carried out with the calibration sample (3) placed, in each detecting step, in a respective different position relative to the analysis instrument (1);
in each detecting step, the detected trend of the fibres is detected at a respective different plurality of points (6) of the working surface (5); and
for each detecting step, the processing step is carried out.

4. The calibration method according to any one of claims 1 to 3, **characterised in that** a plurality of calibration samples (3) is used and, for each calibration sample (3), the detecting step and the processing step are carried out, the calibration samples (3) of the plurality of calibration samples (3) used differing from each other due to a different known trend of the fibres.

5. The calibration method according to any one of claims 1 to 4, **characterised in that** the matrix (15) of the composite material (13) is a polymeric material.

6. The calibration method according to claim 5, **characterised in that** the matrix (15) of the composite material (13) is PLA-based.

7. The calibration method according to claim 5 or 6, **characterised in that** the percentage of fibres (17) by weight in the composite material (13) is between 5% and 40%, preferably between 20% and 30%.

8. The calibration method according to any one of claims 1 to 7, **characterised in that** the fibres (17) of the calibration sample (3) are wood fibres.

9. The calibration method according to any one of claims 1 to 8, **characterised in that** the fibres (17) of the calibration sample (3) are short fibres.

10. The calibration method according to any one of claims 1 to 9, **characterised in that** the composite material (13) has a layered structure comprising a plurality of layers (25), the fibres (17) being divided in the plurality of layers (25) and the working surface (5) being transversal to the plurality of layers (25).

11. The calibration method according to claim 10, **characterised in that** each layer (25) has a relative thickness (29) which has an order of magnitude less than or equal to the order of magnitude of an average length of the fibres (17).

12. The calibration method according to claim 10 or 11, **characterised in that** each layer (25) has a relative thickness (29) less than or equal to 0.2 mm, preferably less than or equal to 0.1 mm.

13. The calibration method according to any one of claims 10 to 12, **characterised in that** each layer (25) has a relative thickness (29) which is substantially constant, the known trend of the fibres being uniform at the entire working surface (5).

14. The calibration method according to any one of claims 1 to 12, **characterised in that** the known trend of the fibres at the working surface (5) is different at a plurality of separate points of the working surface (5).

15. The calibration method according to claim 14 when it depends on claim 10, **characterised in that** one or more layers (25) of the plurality of layers (25) have a relative thickness (29) which, at least at the working surface (5), varies along the extent of said one or more layers (25).

16. The calibration method according to any one of claims 1 to 15, **characterised in that** the calibration sample (3) comprises a frame which supports one or more panels which define the working surface (5) of the calibration sample (3), each panel being constituted of the composite material (13).

17. The calibration method according to any one of claims 1 to 16, **characterised in that** the composite material (13) is an extruded material.
